# EUROPEAN PATENT APPLICATION

(11) **EP 1 669 365 A1**
(43) Date of publication of application: **14.06.2006**
(21) Application number: 04292873.9
(22) Date of filing: 03.12.2004
(51) Int. Cl.: C07H 19/10, C07H 19/20

(54) **Method for preparing enzymatic substrate analogs as inhibitors of bacterial heptosyl-transferases**

(71) Applicant: Mutabilis SA, 93230 Romainville (FR); CENTRE NATIONAL DE LA RECHERCHE SCIENTIFIQUE, 75794 Paris Cedex 16 (FR)
(72) Inventor: Escaich, Sonia, 75012 Paris (FR); Vincent, Stéphane, 5100 Jambes (BE)
(74) Representative: Peaucelle, Chantal

(57) **Abstract**

The invention relates to a A method for making osyl and hexoses derivatives of formula 1, especially 2-fluoro-2-deoxy derivatives. 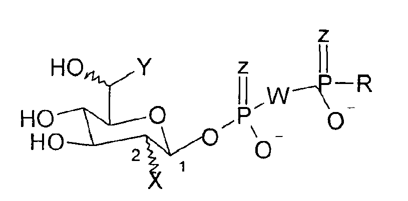 wherein
- R is a nucleoside such as adenosine, cytidine, guanosine, uridine and deoxy analogs such as 2-deoxy,
- X represents OH, halogen, particularly F, NH₂
- Y represents H, CH₂OH, CH₂NH₂, CH₂OPO₃, CH₂OSO₃
- Z represents O or S, and
- W represents O, NH, or CH₂,
said method comprising the steps of:
a) stereoselective fluorophosphorylation of tetrapivaleate 8 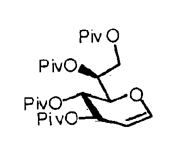 to give β-gluco-type fluorophosphate,
b) hydrogenation and deprotection to give a monophosphate,
c) coupling said glycal with (R)P-morpholidate to give crude sugar nucleotide 1, or alternatively,
d)deacetylation then silylation of heptaglycal 7 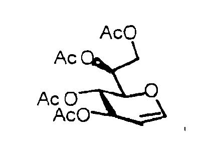 to give tetrasilylated glycal,
e) fluorophosphorylation of said glycal to give β-gluco type fluorophosphate,
f) deprotection of the fluorophosphate and coupling radical R to give 1.

Use of said derivatives as inhibitors of highly virulent proteins of pathogenic bacteria.

## Description

The invention relates to a method for preparing enzymatic substrate analogs as inhibitors of bacterial heptosyltransferases.

It also relates to high throughput methods/assay to measure heptosyl transferase activity of highly virulent protein targets, such as proteins involved in lipopolysaccharide biosynthesis in Gram negative bacteria.

The lipopolysaccharide (LPS short) is a major component of the outer membrane of Gram-negative bacteria. It is composed of three regions: the lipid A, the core oligosaccharide and the O antigen. The core oligosaccharide is divided into the inner core and the outer core. The inner core consists in a moiety of five sugars: two Kdo (Kdo: 3-deoxy-D-manno-octulosonic acid) and three successive heptoses.

During the biosynthesis of the core LPS, the first heptose transfer to Kdo is catalyzed by heptosyltransferase I (protein waaC) and the second heptose transfer by heptosyltransferase II (protein waaF) (Heinrich et al., 1998, Mol. Microbiol. 30:221-232].

Said enzymes represent good anti-virulence target to prevent systemic dissemination of Gram negative pathogens because heptosyl transferases I and II are well conserved in Gram,negative bacteria(FR 02 08 636 in the name of Mutabilis).

The inventors have demonstrated that 2-fluoro-2-deoxy heptosyl derivatives which are substrate analogs are useful inhibitors of LPS biosynthesis and therefore, can be used to prevent and treat infections by said Gram-negative pathogens.

Accordingly, the invention aims to provide a method for preparing such derivatives, and more generally heptosyl and heptose derivatives of formula 1 as disclosed below.

According to another aspect, the invention also relates to means for measuring heptosyl transferase activity.

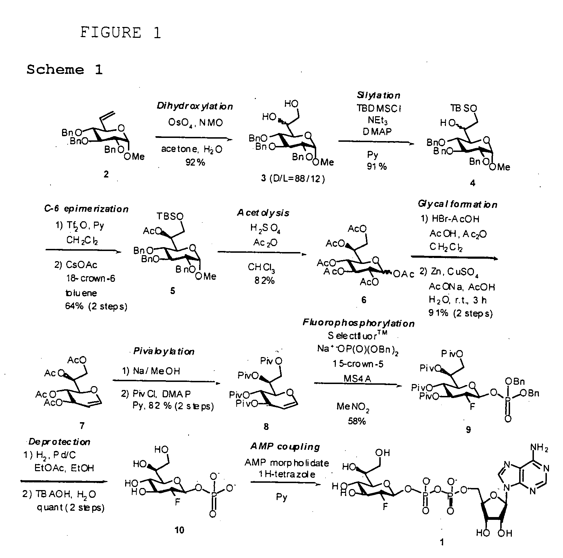

The invention thus relates to a method for making heptosyl and hexoses derivatives of formula 1, especially the 2-fluoro-2-deoxy derivatives, 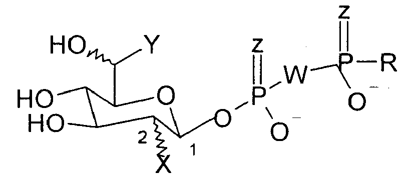 wherein:
- R is a nucleoside such as adenosine, cytosine, guanosine, uridine and deoxy analogs such as 2-deoxy
- X represents OH, halogen, particularly F, NH₂
- Y represents H, CH₂OH, CH₂NH₂, CH₂OPO₃, CH₂OSO₃
- Z represents O or S, and
- W represents O,NH, or CH₂,
said method comprising the steps of:
a) stereoselective fluorophosphorylation of **tetrapivaleate** 8 to give β-gluco-type fluorophosphate 9,
b) hydrogenation and deprotection of 9 to give monophosphate 10,
c) coupling 10 with (R)P-morpholidate to give crude sugar nucleotide 1, or
   alternatively,
d) deacetylation then silylation of heptoglycal 7 to give tetrasilylated glycal 11,
e) fluorophosphorylation of 11 to give β-gluco type fluorophosphate 12,
f) deprotection of 12 to give 10 and coupling radical R to give 1.
   The formulae of said derivatives are given on figure 1.
   Advantageously, said tetrapivaleate 8 is obtained by using the following steps :
g) dihydroxylation of olefin 2 to obtain diol 3 as a mixture of two diastereomers, D and L-glycero-heptoses,
h) regioselective silylation at OH-7 giving 4,
i) triflation at OH-6, and inversion at C6 to give L-glycero type silyl ethers 5L and 5D,
j) removal of the protecting groups of 5 by acetolysis to give per - 0 - acetate 6,
k) treating acetate 6 to give acetylate heptoglycal 7, and
   either
l) converting 7 into tetrapivaleate 8.

In a preferred embodiment of the invention, said derivatives of formula 1 are prepared by carrying out said steps g) to l) followed by said steps a) to f).

Dihydroxylation step g is advantageously carried out with osmium tetroxide and NMO in acetone and water.

This reaction leads to a mixture of two diastereomers D and L-glycero heptoses, with D-glycero sugar as major component.

To obtain the L-glycero type sugar as major component, a regioselective silylation at OH-7 with TBDMSCl, in presence of NEt₃ and DMAP is performed according to step **h** and triflation at OH-6 is carried out by treatment with Tf₂O.

Inversion at C-6 according to step **i** is advantageously carried out with cesium acetate in the presence of 18-crown-6 under ultrasound to afford L-glycero type silylether 5 from 4.

Said ether 5 can be obtained in about 60% overall yield from 4.

The two diastereomers 5L (major) and 5D (minor) can be obtained in a ratio 5L/5D of 88/12 and are easily separated for example by standard silica gel column chromatography.

The removal of all protecting groups of **5** of step **j** is advantageously carried out with excess sulfuric acid in chloroform and acetic anhydride.

*Per-O*-acetate **6** can be obtained in 82% yield.

The glycal formation of step **k** is particularly performed by subjecting acetate **6** to bromination, followed by treating with zinc powder, copper sulfate, and sodium acetate in acetic acid and water.An acetylated heptoglycal **7** can be obtained with yields over 90%.

According to step **1**, heptoglycal **7** is converted into tetrapivaloate 8 by Zemplen's deacetylation and *per-O-*pivaloylation with pivaloyl chloride. Yields above 82 % are obtainable.

Stereoselective *fluorophosphorylation* is performed according to step **a** by treating tetrapivaloate **8** with Selectfluor™ (triflate form), sodium dibenzylphosphate, and 15-crown-5 in nitromethane to give β-gluco-type fluorophosphate **9.** Yields of about 60% can be obtained.

Pivaloylated β -fluorophosphate **9** is subjected to hydrogenation, then depivaloylation by tetrabutylammonium hydroxide to afford monophosphate 10 in quantitative yield according to step **b**.

Finally, the coupling step **c** is carried out by reacting monophosphate 10 with (R)P-morpholidate in the presence of 1H-tetrazole in pyridine at room temperature to give crude sugar-nucleotide **1**.

The conversion into **1** was measured to be 90% by ¹H- and ³¹P-NMR.

Alternatively, **1** is synthesized from **7**. According to step **d**, heptoglycocal 7 is deacetylated following a standard Zemplen's procedure then *silylated* to furnish tetrasilylated glycal 11 in yield above 90%.

The fluorophosphorylation of **11** gives the β -gluco-type fluorophosphate 12, in yield above 40%, along with its isomeric α-gluco-type fluorophosphate (25% yield).

Fluorophosphate **12** is deprotected according to step **f** by hydrogenation and treatment with TBAF in THF to afford monophosphate 10 in yield above 50%.

Preferably R is adenosine.

Alternatively, the method of the invention for making 2-fluoro-D-glyceroheptose derivatives of **1** is a biomimetic chemo-enzymatic synthesis.

Adenosine-diphosphate-mannoheptose (ADPH), the substrate of the heptosyltransferases WaaC and WaaF, derives from D-glycero-D-manno-heptose- β -1-phosphate.

This glycosyl phosphate is transformed into a nucleotide-sugar through an enzymatic AMP transfer (catalyzed by RfaE) followed by an epimerization at the C6 position of the heptose (Valvano et al., 2000, Kneidinger et al., 2001; and Kneidinger et al. 2002)

The last step is catalyzed by the epimerase WaaD.

According to the invention, fluorinated analogs of D-glycero-D-manno-heptose-β-1-phosphate, such as the fluorophosphate **18** (scheme 3), are used as a substrate of RfaE: this reaction leads to the formation of an intermediate ADP-fluoroheptose.

As this biosynthetic sequence might occur *in vivo,* molecule **18** is a pro-inhibitor of the two transferases WaaC and WaaF.

The synthesis of **18** (depicted in scheme 3) follows the synthetic strategy developped for its epimer **10** from a common intermediate **3** (protections at C6 and C7, acetolysis, glycal formation, fluorophosphorylation of a silylated intermediate and deprotections).

The invention particularly relates to the obtention of derivatives of formulae A, B, C, D and E. 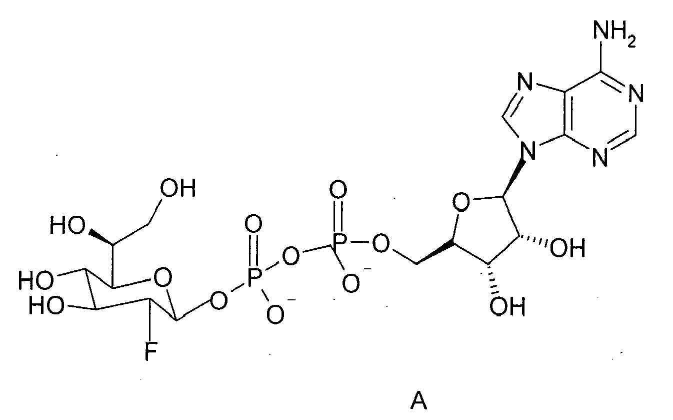 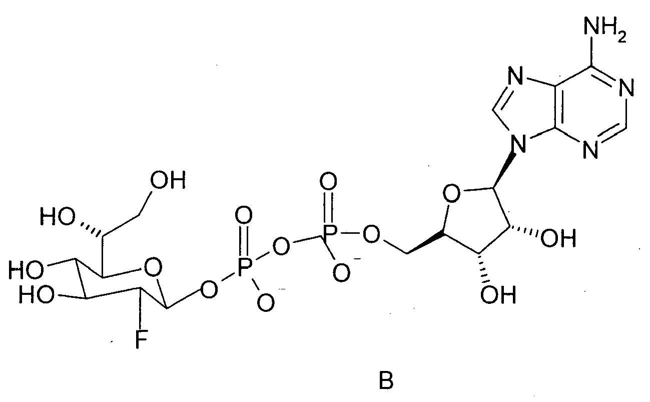 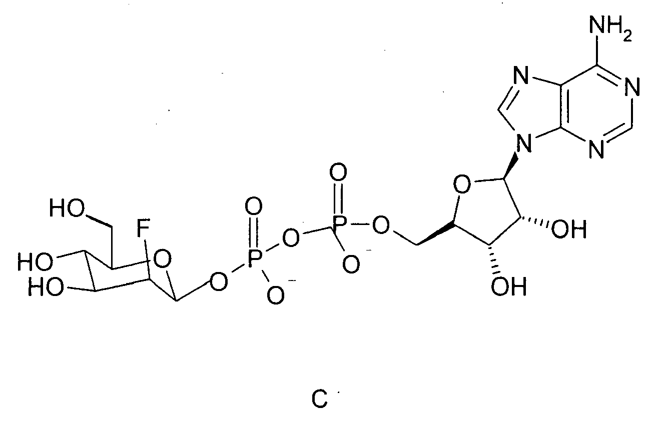 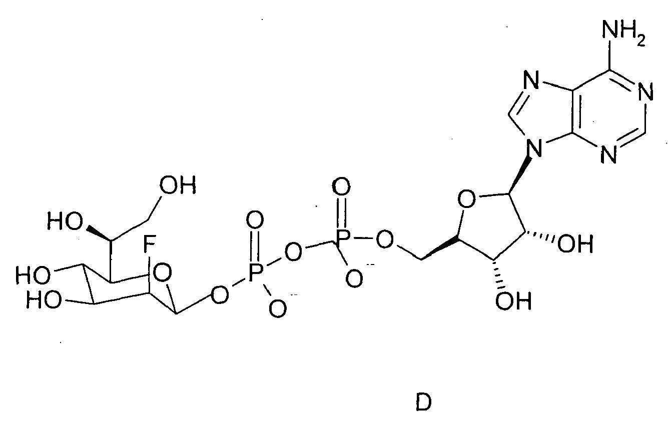 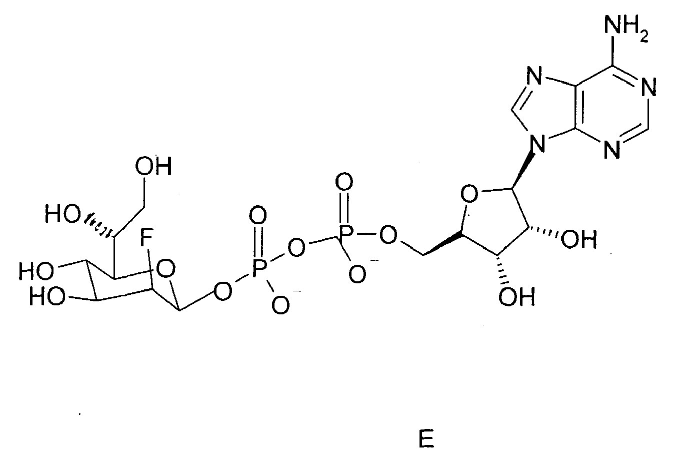 and alpha corresponding anomers.

The derivatives of formula 1 wherein Y represents CH₂OH, X represents F, and the other substituents have the above defined meanings, are new compounds. Accordingly, they are also part of the invention.

The invention also relates to the use of a derivative such as obtained by the above defined method, as inhibitors of heptosyl transferase proteins of pathogenic bacteria.

It notably relates to the use of the ADP-L-glycero-α-D-manno-heptose (ADP-2F heptose) as inhibitor of WaaC.

According to another aspect, the invention also relates to a quantitative assay to assess the enzymatic activity of identified targets. It more particularly relates to a quantitative assay for determining heptosyltransferase activity of target proteins of pathogenic bacteria, comprising incubating, as donor substrate, an ose derivative such as obtained by the above defined method, particulary ADP-L-glycero-α-D-manno-heptose and, as acceptor substrate, Re-LPS, composed of lipid A with two Kdo, and measuring the reaction products of the transferase activity using a fluorometric assay.

The invention thus provides means to measure the heptosyltranferase activity for example of both proteins WaaC and WaaF.

Other characteristics and advantages of the invention will be given in the examples and the figures, which represent,
Figure 1 : reaction schemes 1, 2, 3 and 4.
Figure 2 : the % of inhibition ADP - 2F - heptose

### Experimental Section

**General Techniques**. All reactions were carried out under argon atmosphere. Yields refer to chromatographically and spectroscopically homogeneous materials. Tetrahydrofuran, diethyl ether, and toluene were freshly dried over sodium benzophenone, dichloromethane over P₂O₅, and acetonitrile and nitromethane over CaH₂. Reagents were purchased at the highest commercial quality from Sigma, Aldrich, or Fluka, and used without further purification. All reactions were monitored by thin-layer chromatography (TLC) carried out on Merck aluminum roll silica gel 60-F₂₅₄ using UV light and ethanoic phosphomolybdic acid or ethanoic sulfuric acid for visualization. ¹H, ¹³C, ¹⁹F, and ³¹P NMR spectra were recorded with a Bruker AC-250 and AMX-400 spectrometer. All compounds were characterized by ¹H, ¹³C, ¹⁹F, and ³¹P NMR as well as by ¹H-¹H and ¹H-¹³C correlation experiments. The following abbreviations were used to describe the multiplicities: s = singlet, d = doublet, t = triplet, m = multiplet, br = broad, bs = broad singlet. Merck silica gel (60, particle size 0.040-0.063 mm) was employed for flash column chromatography using cyclohexane-ethyl acetate as eluting solvents. Size exclusion chromatographies were performed with a Pharmacia Biotech Äkta FPLC apparatus on Sephadex G15 column (2.5/60 cm), and the fractions containing adenine derivatives were detected by UV. Purifications of nucleotide-sugars were realized by semi-preparative HPLC using a Waters Delta prep 4000 chromatography system equipped with a NovaPack C18 column (eluent: triethylammonium acetate 50 mM, pH = 6.8).

### Dihydroxylation (Scheme 1)

**Methyl 2,3,4-Tri-*O*-benzyl-α-D-heptoglucopyranoside** (**3).** To a solution of olefin 2 (2.18 g, 4.72 mmol) and NMO (1.27 g, 9.44 mmol) in acetone (25 mL) and water (3 mL) was added OsO₄ (2.5% in tBuOH, 1.82 mL). After 13 h stirring at room temperature, the reaction mixture was poured into 5% Na₂S₂O₃ aq. (20 mL) and stirred for 30 min. The aqueous phase was successively extracted by dichloromethane three times. The combined organic layers were washed with saturated aqueous solution of NaHCO₃ then brine, dried over MgSO₄, and concentrated *in vacuo*. The residue was purified by flash chromatography (silica gel, cyclohexane/ethyl acetate 3:1) to give diol 3 as a colorless syrup (2.73 g, 92%). This molecule has been previously described in the literature.

### Silylation (Scheme 1)

**Methyl 2,3,4-tri-*O*-benzyl-7-*O*-TBDMS-α-D-eptoglucopyranoside** (4). To a solution of diol 3 (200 mg, 0.404 mmol), DMAP (4.9 mg, 0.040 mmol), and triethylamine (165 µL, 1.21 mmol) in pyridine (35 mL) was gradually added TBDMSC1 (94 mg, 0.607 mmol) at 0°C. After 13 h stirring at room temperature, the reaction mixture was poured into saturated aqueous solution of NaHCO₃ and the aqueous phase was extracted by dichloromethane. The combined organic layers were washed with saturated aqueous solution of NaHCO₃ then brine, dried over MgSO₄, and concentrated *in vacuo*. The residue was purified by flash chromatography (silica gel, cyclohexane/ethyl acetate 5:1) to furnish silyl ether 4 as a colorless syrup (224 mg, 91%).

### C-6 epimerization (Scheme 1)

**Methyl 6-*O*-acetyl-2,3,4-tri-*O*-benzyl-7-*O*-TBDMS-6-L-*glycero-*α-D-heptoglucopyranoside (5).** To a solution of silyl ether **4** (234 mg, 0.384 mmol) in pyridine (10 mL) was added trifluoromethanesulfonic anhydride (188 µL, 1.15 mmol) dropwise at -40°C, then the reaction mixture was allowed to warm to 0°C and stirred for 1h.

After concentration *in vacuo*, the residue was chromatographed on silica gel (cyclohexane/ethyl acetate 5:1), and all the fractions were successively concentrated and dried *in vacuo*. The resulting triflate was dissolved into toluene (10 mL), and to this solution was added cesium acetate (223 mg, 1.16 mmol) and 18-crown-6 (305mg, 1.16 mmol). The reaction mixture was sonicated for 6 h at room temperature, and diluted into ethyl acetate. The organic layer was washed with brine, dried over MgSO₄, and concentrated *in vacuo*. The residue was purified by flash chromatography (silica gel, cyclohexane/ethyl acetate 8:1) to give acetate 5 as a colorless syrup (160 mg, 64%): [α]²³_{D} -4.0 (c 0.5, CHCl₃); ¹H NMR (400MHz, CDCl₃): δ 7.40 (m, 15 H, ArH), 5.43 (t 1H, *J*_{5,6} = *J*_{6,7} = 7.1 Hz, H-6), 4.53-5.09 (m, 6H, ArCH₂), 4.74 (d, 1H, *J*_{1,2} = 3.6 Hz, H-1), 4.11 (*J*_{2,3} = *J*_{3,4} = 9.2 Hz, H-3), 4.04 (br-d, 1H, *J*_{4,5} = *J*_{5,6} = 10.1 Hz, H-5), 3.80 (m, 2H, H-7), 3.62 (dd, 1H, *J*_{1,2} = 3.5 Hz, *J*_{2,3} = 9.7 Hz, H-2), 3.49 (dd, 1H, *J*_{3,4} = 9.8 Hz, *J*_{4,5} = 9.0 Hz, H-4), 3.46 (s, 3H, OMe), 0.94 (s, 9H, SitBu), 0.11 and 0.13 (sx2, 6H, SiMe); ¹³C NMR (101MHz, CDCl₃): δ 170.0, 138.5, 138.0, 137.8, 128.4, 128.3, 128.2, 128.0x2, 127.9, 127.7, 127.6, 97.9, 82.3, 79.6, 76.9, 75.8, 75.1, 73.1, 70.7, 67.2, 60.0, 55.1, 25.7, 20.9, 18.0, -5.4, -5.6; MS(DCI): m/z 668 (M+NH₄); HRMS calcd for C₃₇H₅₄O₈NSi (MNH₄⁺): 668.3619. Found: 668.3628.

### Acetolysis (Scheme 1)

**1,2,3,4,6,7-Hexa-*O*-acetyl-6-L-*glycero*-D-heptoglucopyranose** (6). To a solution of silyl ether 5 (3.91 g, 5.99 mmol) and acetic anhydride (7.53 mL) in chloroform (4.89 mL) was added conc. H₂SO₄ (2.93 mL) dropwise at 0°C. The reaction mixture was allowed to warm to room temperature and stirred for 12 h. After addition of potassium carbonate, the solution was poured into water and extracted by dichloromethane three times. The combined organic layers were washed with saturated aqueous solution of NaHCO₃ then brine, dried over MgSO₄, and concentrated *in vacuo*. The residue was purified by flash chromatography (silica gel, cyclohexane/ethyl acetate 3:1 then 1:1) to give acetate **6** as a colorless syrup (2.27 g, 82%). The α/β ratio was 1/0.14: ¹H NMR (400MHz, CDCl₃): δ 6.34 (d, *J*_{1,2} = 3.8 Hz, H-1α), 5.66 (d, *J*_{1,2} = 8.3 Hz, H-1β), 5.46 (d, *J*_{2,3} = *J*_{3,4} = 10.0 Hz, H-3α), 5.25 (ddd, *J*_{5,6} = 2.1 Hz, *J*_{6,7} = 5.2 and 7.2 Hz, H-6α), 5.21 (dd, *J*_{1,2} = 3.6 Hz, *J*_{2,3} = 9.1 Hz, H-2α), 5.10 (t, *J*_{3,4} = *J*_{4,5} = 9.8 Hz, H-4α), 4.27 (dd, *J*_{6,7} = 5.0 Hz, *J*_{gem} = 11.7 Hz, H-7α), 4.19 (dd, *J*_{4,5} = 10.2 Hz, *J*_{5,6} = 2.1 Hz, H-5α), 4.15 (dd, *J*_{6,7} = 7.6 Hz, *J*_{gem} = 11.7 Hz, H-7α), 2.02-2.19 (m, COCH₃); ¹³C NMR (101MHz, CDCl₃): δ 170.4, 170.1, 169.6, 169.3, 168.5, 92.0 (C-1β), 88.7 (C-1α), 70.0, 69.9, 69.0, 66.9, 66.5, 62.1, 20.7, 20.6x2, 20.4, 20.3; MS(DCI): m/z 480 (M+NH₄) . HRMS calcd for C₁₉H₃₀O₁₃N (MNH₄⁺): 480.1717. Found: 480.1711.

### L-Glycal formation (Scheme 1)

**3,4,6,7-Tetra-*O*-acetyl-6-L-*glycero*-D-heptosylglycal(7).**To a solution of per-O-acetylated L-glycero-heptopyranose **6** (2.17 g, 4.69 mmol), acetic anhydride (1.32 mL), and acetic acid (1.12 mL) in dichloromethane (4 mL) was added HBr-AcOH (9.68 mL, 56.3 mmol), and the reaction mixture was stirred for 12 h at room temperature. The reaction was quenched by addition of sodium acetate (4.61 g, 56.3 mmol). The suspension was transferred into a suspension of CuSO₄ (186 mg, 1.17 mmol), zinc powder (11.7 g, 178 mmol), and sodium acetate (8.78 g, 113 mmol) in water (9.2 mL) and acetic acid (13.8 mL). The mixture was vigorously stirred for 4 h at room temperature then filtered through a pad of Celite, which was washed with ethyl acetate and water. The organic layer of the filtrate was washed with saturated aqueous solution of NaHCO₃ then brine, dried over MgSO₄, and concentrated *in vacuo*. The residue was purified by flash chromatography (silica gel, cyclohexane/ethyl acetate 3:1) to furnish a glycal 7 as a colorless syrup (1.47 g, 91%): [α]²³_{D} -13.8 (c 0.5, CHCl₃); ¹H NMR (400MHz, CDCl₃) : δ 6.44 (dd, 1H, *J*_{1,2} = 6.2 Hz, *J*_{1,3} = 1.6 Hz, H-1) , 5. 48 (ddd, 1H, *J*_{1,3} = 1.8 Hz, *J*_{2,3} = 2. 4 Hz, *J*_{3,4} = 7.2 Hz, H-3), 5.38 (ddd, 1H, *J*_{5,6} = Hz, *J*_{6,7} = Hz, H-6), 5.25 (dd, 1H, *J*_{3,4} = 7.3 Hz, *J*_{4,5} = 9.9 Hz, H-4), 4.79 (dd, 1H, *J*_{1,2} = 6.2 Hz, *J*_{2,3} = 2. 4 Hz, H=2), 4.35 (dd, 1H, *J*_{6,7} = 4.8 Hz, *J*_{gem} = 11.7 Hz, H-7), 4.19 (m, 2H, H-5 and H-7), 2.03-2.09 (sx4, 12H, COCH₃); ¹³C NMR (101MHz, CDCl₃) : δ 170.6, 170.4, 170.2, 169.6, 145.5, 100.0, 74.6, 69.4, 66.6, 66.1, 62.3, 20.9, 20.6; MS (DCI) : m/z 362 (M+NH₄) ; Elemental analysis calcd for C₁₅H₂₀O₉: C, 52.32; H, 5.85. found: C, 52.32; H, 5.84.

### Pivaloylation (Scheme 1)

**3,4,6,7-Tetra-*O*-pivaloyl-6-L-*glycero*-D-heptosylglycal (8).** To a suspension of acetate 7 (488 mg, 1.42 mmol) in MeOH (5 mL) was added sodium (10 mg), and the reaction mixture was stirred for 2 h. The solution was concentrated *in vacuo*, and the residue was dissolved in pyridine (20 mL). To the solution, pivaloyl chloride (2.10 mL, 17.0 mmol) was added dropwise at 0°C, and the mixture was allowed to warm to room temperature. After 12 h of stirring at room temperature under argon, cold saturated aq. NaHCO₃ was added, and the mixture was extracted twice with dichloromethane. The combined organic layers were washed with brine, dried over MgSO₄, and concentrated *in vacuo.* The residue was purified by flash chromatography (silica gel, hexane/ethyl acetate 10:1) to afford pivaloate 8 as a white powder (598 mg, 82%): [α]²³_{D} - 17.6 (c 0.5, CHCl₃); ¹H NMR (400MHz, CDCl₃): δ 6.46 (d, 1H, *J*_{1,2} = 6.0 Hz, *J*_{1,3} = 1.4 Hz, H-1), 5.44 (ddd, 1H, *J*_{1,3} = 1.6 Hz, *J*_{2,3} = 2.3 Hz, *J*_{3,4} = 7.0 Hz, H-3), 5.40 (ddd, 1H, *J*_{5,6} = 2.9 Hz, *J*_{6,7} = 4.3 and 7.4 Hz, H-6), 5.28 (dd, 1H, *J*_{3,4} = 7.0 Hz, *J*_{4,5} = 9.5 Hz, H-4), 4.79 (dd, 1H, *J*_{1,2} = 6.0 Hz, *J*_{2,3} = 2.5 Hz, H-2), 4.38 (dd, 1H, *J*_{6,7} = 4.4 Hz, *J*_{gem} = 11.7 Hz, H-7), 4.23 (dd, 1H, *J*_{4,5} = 9. 6 Hz, *J*_{5,6} = 2. 9 Hz, H-5), 4.20 (dd, 1H, *J*_{6,7} = 7.4 Hz, *J*_{gem} = 11.6 Hz, H-7), 1.18-1.26 (sx4, 36H, CO^{*t*}Bu); ¹³C NMR (101 MHz, CDCl₃): δ 178.0, 177.9, 177.0, 176.2, 145.5, 99.9, 75.2, 69.2, 66.2, 65.5, 62. 9, 38.7x2, 27.1, 27.0x2, 26. 9; MS (DCI) : m/z 530 (M+NH₄); HRMS calcd for C₂₇H₄₈O₉N (MNH₄⁺): 530.3329. Found: 530.3336.

### Stepwise fluorophosphorylation (Scheme 1)

**2-Deoxy-2-fluoro-3,4,6,7-tetra-*O*-pivaloyl-6-L-*glycero*-β-1-(dibenzylphosphoryl)-D-hepto-glucopyranose (9).** A mixture of pivaloate **8** (543 mg, 1.06 mmol) and MS4A (200 mg) in nitromethane (15 mL) was stirred for 2 h at room temperature under argon atmosphere. Selectfluor-(TfO)₂ (761 mg, 1.59 mmol) was added very quickly at 0°C, and the reaction mixture was allowed to warm to room temperature. After 12 h of stirring, sodium dibenzylphosphate (955 mg, 3.18 mmol) and 15-crown-5 (700 mg, 3.18 mmol) was added with flowing argon gas, and the mixture was heated to 60°C and stirred for 2 h. The suspension was filtered through a pad of Celite, and the pad was washed well with dichloromethane. The filtrate was concentrated in vacuo, and the residue was chromatographed on silica gel (hexane/ethyl acetate 5:1) to give fluorophosphate 9 (497mg, 58 %) as a colorless syrup: [α]²³_{D} -1.7 (c 0.5, CHCl₃); ¹H NMR (400MHz, CDCl₃): δ 7.38 (m, 10 H, ArH), 5.43 (ddd, 1H, *J*_{1,2} = 7.7 Hz, *J*_{1,F} = 2.8 Hz, H-1), 5.38 (dt, 1H, *J*_{2,3} = *J*_{3,4} = 9.3 Hz, *J*_{3,F} = 13.3 Hz, H-3), 5.26 (dddd, 1H, *J*_{5,6} = 1.9 Hz, *J*_{6,7} = 4.6 and 8.0 Hz, H-6), 5.06-5.18 (m, 4H, ArCH₂), 5.11 (t, 1H, *J*_{3,4} = *J*_{4,5} = 9.8 Hz, H-4), 4.38 and 4.51 (ddx2, *J*_{1,2} = 7.8 Hz, *J*_{2,3} = 9.0 Hz, *J*_{2,F} = 50.6 Hz, H-2), 4.28 (dd, 1H, *J*_{6,7} = 4.7 Hz, *J*_{gem} = 11.7 Hz, H-7), 4.04 (dd, *J*_{6,7} = 8.1 Hz, *J*_{gem} = 11.6 Hz, H-7), 3.91 (dd, 1H, *J*_{4.5} = 10.1 Hz, *J*_{5,6} = 2.0 Hz, H-5), 1.18-1.25 (sx4, 12H, ^{*t*}Bu); ¹³C NMR (101MHz, CDCl₃): δ 177.7, 177.0, 176.7, 176.0, 135.2, 135.1, 128.6x2, 128.0, 127.9, 96.4, 96.3, 96.1x2, 90.6, 90.5, 88.7, 88.6, 73.6, 72.1, 71.9, 69.7x2, 69.6x2, 65.8, 65.6x2, 62.7, 38.8, 38.7x2, 38.6, 30.1, 27.1, 26.9x3; ¹⁹F NMR (235MHz, D₂O): δ-199.2 (dd, *J*_{2,F} = 51.7 Hz, *J*_{3,F} = 14.1 Hz); ³¹P NMR (101MHz, D₂O): δ-2.93 (s); MS(DCI): m/z 826 (M+NH₄); HRMS calcd for C₄₁H₅₉O₁₃FP (MH⁺): 809.3677. Found: 809.3682.

### Deprotection (Scheme 1)

**2-Deoxy-2-fluoro-6-L-*glycero*-β-1-phosphoryl-D-heptoglucopy ranose di-tributylammonium salt (10).** To a suspension of fluorophosphate 9 (125 mg, 0.155 mmol) and Pd/C (62 mg) in ethyl acetate (1 mL) and ethanol (2 mL) was added triethylamine (42 µL, 0.309 mmol). The mixture was hydrogenated under H₂ atmosphere (1 bar) for 12 h, and filtered through a pad of Celite. The filtrate was concentrated in vacuo to afford a triethylammonium salt, that was dissolved in water (1 mL). To this solution was added tetrabutylammonium hydroxide (40% in H₂O, 1.34 mL, 2.07 mmol), and the mixture was stirred at room temperature for 26 h. After neutralization by IR-120A (H⁺) resin, the solution was filtered and concentrated in vacuo. The residue was dissolved in ethanol (4 mL), and to the solution was added tributylamine (74 µL, 0.310 mmol). Concentration of the mixture in vacuo afforded a monophosphate 10 (112 mg, quantitative yield) as a syrup. : [α]²³_{D} -1.7 (c 0.5, H₂O); ¹H NMR (250 MHz, D₂O): δ 4.97 (ddd, 1H, *J*_{1,2} = 7.7 Hz, *J*_{1,F} = 2.6 Hz, H-1), 3.91 and 4.12 (tx2, 1H, *J*_{1,2} = *J*_{2,3} = 8.0 Hz, *J*_{2,F} = 51.0 Hz, H-2) , 3.34-3.81 (m, 6H, H-3, H-4, H-5, H-6, and H-7); ¹³C NMR (63 MHz, D₂O): δ 94.5, 94.4, 94.1, 94.0, 93.8, 93.7, 90.8, 90.7, 74.2, 73.9, 72.8, 71.3, 71.2, 70.6, 64.5; ¹⁹F NMR (235MHz, D₂O): δ -199.2 (dd, *J*_{2,F} = 51. 7 Hz, *J*_{3,F} = 14.1 Hz); ³¹P NMR (101MHz, D₂O): δ 0.83 (s); HRMS calcd for C₇H₁₃O₉FP (MH⁺) 291.0214. Found 291.0283.

### Silylation of L-heptoglycal (Scheme 2)

**3,4,6,7-Tetra-*O*-TBDMS-6-L-*glycero*-D-heptosylglycal(11).** To a suspension of acetate **7** (488 mg, 1.42 mmol) in MeOH (5 mL) was added sodium (10 mg), and the reaction mixture was stirred for 2 h. The solution was concentrated *in vacuo*, and the residue was dissolved in *N,N*-dimethylformamide (20 mL). To a suspension was added imidazole (2.83 g, 41.6 mmol) then TBDMSCl (4.70 g, 31.1 mmol) at 0°C. After 12 h of stirring at room temperature, the solution was poured into brine and the aqueous phase was extracted by ethyl acetate three times. The combined organic layers were washed with saturated aqueous solution of NaHCO₃ then brine, dried over MgSO₄, and concentrated *in vacuo*. Purification of the residue by flash chromatography (silica gel, cyclohexane/ethyl acetate 3:1 then 1:1) gave a tetrasilylated glycal **11** as a white powder (1.56 g, 95%): [α]²³_{D} -21.1 (c 0.5, CHCl₃); ¹H NMR (400MHz, CDCl₃): δ 6.37 (d, 1H, *J*_{1,2} = 6.4 Hz, H-1), 4.75 (td, 1H, *J*_{1,2} = 6.4 Hz, H-2), 4.22 (br, 2H, H-5 and H-6), 4.03 (br-d, 1H, H-4), 3.71 and 3.96 (ddx2, 2H, H-7 _{R,S}), 3.87 (br, 1H, H-3), 0.91-0.94 (sx4, 36H, Si^{*t*}Bu), 0.06-0.14 (sx7, 24H, SiMe); ¹³C NMR (101MHz, CDCl₃): δ 143.4, 100.5, 79.4, 69.4, 69.3, 66.4, 65.9, 26.9, 26.2, 26.0, 25.8x2, 18.6, 18.2, 18.0, 17.9, -4.3, -4.4x3, -4.6x2, -5.1, -5.5; MS(DCI): m/z 650 (M+NH₄); HRMS calcd for C₃₁H₆₈O₅Si₄Na (MNa⁺) : 655.4041. Found: 655.4057.

### Nonstepwise fluorophosphorylation of silylated L-glycal (Scheme 2)

### 2-Deoxy-2-fluoro-3,4,6,7-tetra-O-TBDMS-6-L-glycero-β-1 (di benzyphosphoryl)-D-hepto-glucopyranoside (12) .

A mixture of tetrasilylated glycal 11 (342 mg, 0.540 mmol), sodium dibenzylphosphate (811 mg, 2.70 mmol), 15-crown-5 (594 mg, 2.70 mmol), and MS4Å (100 mg) in nitromethane (15 mL) was stirred for 2 h at room temperature under argon atmosphere. Selectfluor- (TfO)₂ (517 mg, 1.08 mmol) was added very quickly, and the mixture was heated to 60□C and stirred (1 equiv of Selectfluor-(TfO)₂ was added every 1 hour until the consumption of the glycal, total 5 equiv). After disappearance of an intermediate, the suspension was filtered through a pad of Celite, and the pad was washed well with dichloromethane. The filtrate was concentrated in vacuo, and the residue was chromatographed on silica gel (toluene then cyclohexane/ethyl acetate 10:1) to give fluorophosphate **12** (226 mg, 45 %) as a colorless syrup: [α]²³_{D} +2.6 (c 0.5, CHCl₃); ¹H NMR (400MHz, CDCl₃): δ 7.37 (m, 10H, ArH), 5.41 (ddd, 1H, *J*_{1,2} = 7.5 Hz, *J*_{1,F} = 3.2 Hz, *J*_{1,P} = 7.5 Hz, H-1), 5.01-5.17 (m, 4H, ArCH₂), 4.15 and 4.18 (tx2, 1H, *J*_{1,2} = 7.5 Hz, *J*_{2,3} = 7.7 Hz, *J*_{2,F} = 50.7 Hz, H-2), 4.06 (ddd, 1H, *J*_{5,6} = 1.6 Hz, *J*_{6,7} = 5.5 and 7.4 Hz, H-6), 3.93 and 3.97 (tx2, 1H, *J*_{2,3} = 7.7 Hz, *J*_{3,4} = 8.0 Hz, *J*_{3,F} = 16.0 Hz, H-3) , 3.89 (t, 1H, *J*_{3,4} = *J*_{4,5} = 8.0 Hz, H-4), 3.72 (dd, 1H, *J*_{6,7} *=* 7.7 Hz, *J*_{gem} = 9.7 Hz, H-7), 3.59 (m, 2H, H-5 and H-7), 0.90-0.96 (sx4, 36H, Si^{t}Bu), 0.04-0.19 (sx7, 24H, SiMe); ¹³C NMR (101MHz, CDCl₃): δ 128.5, 128.4x2, 127.9, 127.8, 96.4x2, 96.2x2, 93.5, 93.4, 91.6, 91.5, 77.3, 77.1, 76.7, 72.0, 71.9, 71.3, 69.4, 69.3x3, 26.2, 26.0, 25.9x2, -2.5, -3.2, -3.3, -3.4x2, -3.5, -3.7, -4.0; ¹⁹F NMR (235MHz, CDCl₃): δ 191.5 (dd, *J*_{2,F} = 50.0 Hz, *J*_{3,F} = 14.0 Hz) ; ³¹P NMR (101MHz, CDCl₃): δ -2.79 (s); MS(DCI): m/z 946 (M+NH₄); HRMS calcd for C₄₅H₈₆O₉NFSi₄P (MNH₄⁺) : 946.5101. Found: 946.5095.

### Deprotection of silylated L-fluorophosphate (Scheme 2)

**2-Deoxy-2-fluoro-6-L-*glycero*-β-1-phosphoryl-D-heptoglucopy ranose di-tributylammonium salt (10).** To a suspension of fluorophosphate 12 (141 mg, 0.152 mmol) and Pd/C (40 mg) in ethanol (10 mL) was added triethylamine (31 µL, 0.228 mmol). The mixture was hydrogenated under H₂ atmosphere for 1 h, and filtered through a pad of Celite. The filtrate was concentrated in vacuo to afford a triethylammonium salt, that was dissolved in tetrahydrofuran (10 mL). To this solution was added tetrabutylammonium fluoride (206 mg, 0.654 mmol), and the mixture was stirred at room temperature for 1.5 h. After concentration in vacuo, to the residual syrup was added water (5mL), thus generating a colorless, precipitate. The precipitate was removed by filtration, and the aqueous solution was concentrated in vacuo. The resulting yellow syrup was dissolved in water (1mL), and passed through a Dowex 50Wx8 column (Na⁺ form, 2x20 cm). This solution was concentrated, the residue was dissolved in water (1mL) and passed through a Dowex 50Wx8 column (Bu₃N form, 1x15 cm), and concentrated in vacuo to afford a monophosphate 10 (76.3 mg, 76%) as a syrup. All analytical data exactly corresponded to the ones obtained by the previously described procedure (Deprotection, Scheme 1).

### AMP coupling (Scheme 1 and Scheme 2)

**Adenosine 5'-diphospho-2-Deoxy-2-fluoro-6-L-*glycero*-β-D-heptoglucopyranoside(1).** Monophosphate **10** (50 mg, 0.075 mmol) was azeotroped with dry pyridine three times under argon atmosphere. To the residue was added AMP morpholidate (214 mg, 0.302 mmol) and 1H-tetrazole (42 mg, 0.603 mmol), and the mixture was well-dried under vacuum for 24 h. Dry pyridine (2mL) was added, and the mixture was stirred under argon atmosphere for 52 h. After concentration of the mixture, the residue was decantated with ethyl acetate. The resulting solid part was purified on HPLC (preparative C-18 coated silica gel) using triethylammonium acetate buffer (pH 6.8) with 2% acetonitrile to give a mixture of sugar nucleotide 1 and AMP dimer. The mixture was separated on Sephadex G-15 gel (water) to furnish sugar nucleotide 1 as a syrup. Repeated lyophilizations of the syrupy product afforded pure sugar nucleotide 1 as solid (3.2mg, 13 %): ¹H NMR (400 MHz, D₂O): δ 8.25 and 8.49 (sx2, 2H, ArH), 6.13 (d, 1H, *J*_{1',2'} = 6.0 Hz, H-1'), 5.21 (ddd, 1H, *J*_{1,2} = 7.8 Hz, *J*_{1,F} = 2.6 Hz, H-1), 4.75 (m, 1H, H-2'), 4.52 (dd, 1H, *J*_{2',3'} = 5.1 Hz, *J*_{3',4'} = 3.5 Hz, H-3'), 4.39 (t, 1H, *J*_{3',4'} = *J*_{4',5'} = 3.1 Hz, H-4'), 4.21 (dd, 1H, *J*_{4',5'} = 3.2 Hz, *J*_{gem} = 5.2 Hz, H-5'), 4.08 and 4.21 (ddd, 1H, *J*_{1,2} = 7.9 Hz, *J*_{2,3} = 8.7 Hz, *J*_{2,F} = 50.7 Hz, H-3'), 3.91 (dt, 1H, *J*_{5,6} = 1.3 Hz, *J*_{6,7} = 6.9 and 7.9 Hz, H-6), 3.78 (dd, 1H, *J*_{2,3} = *J*_{3,4} = 9.2 Hz, *J*_{3,F} = 15.0 Hz, H-3'), 3.69 (dd, 1H, *J*_{6,7} = 7.1 Hz, *J*_{gem} = 11.6 Hz, H-7), 3.68 (dd, 1H, *J*_{6,7} = 7.8 Hz, *J*_{gem} = 11.6 Hz, H-7), 3.63 (t, 1H, *J*_{3,4} = *J*_{4,5} = 9.5 Hz, H-4), 3.48 (dd, 1H, *J*_{4,5} = 9.8 Hz, *J*_{1.5} = 1.5 Hz, H-5); ¹³C NMR (101 MHz, D₂O): δ 156.0, 153.3, 95.8x2, 95.6, 95.5, 93.3, 87.2, 84.3, 84.2, 75.1, 74.6, 74.4, 74.2, 70.8, 68.7, 68.6, 68.5, 65.7, 65.6, 62.4; ¹⁹F NMR (235MHz, D₂O): δ -199.9 (dd, *J*_{2,F} = 50.8 Hz, *J*_{3,F} = 13.9 Hz); ³¹P NMR (101MHz, D₂O): δ -11.3 and -13.3 (d, *J* = 222 Hz); ESIMS calcd for C₇H₁₃O₉FP (M-2H⁺Na) 642.06. Found 642.07.

### Protections and epimer separation (Scheme 3)

**Methyl 6-*O*-acetyl-2,3,4-tri-*O*-benzyl-7-*O*-TBDMS-6-D-*glycero-*α-D-heptoglucopyrano-side (13).** To a solution of diol 3 (650 mg, 1.31 mmol), DMAP (15.9 mg, 0.130 mmol), and triethylamine (536 µL, 3.93 mmol) in pyridine (40 mL) was gradually added TBDMSCl (306 mg, 1.97 mmol) at 0°C. After 10h stirring at room temperature, the reaction mixture was poured into saturated aqueous solution of NaHCO₃ and the aqueous phase was extracted by dichloromethane. The combined organic layers were washed with saturated aqueous solution of NaHCO₃ then brine, dried over MgSO₄, and concentrated *in vacuo*. The residue was dissolved in pyridine (30mL), and the mixture was added acetic anhydride (15mL). After 18 h stirring at room temperature, the reaction mixture was poured into water, and the aqueous phase was extracted by dichloromethane. The combined organic layers were washed with saturated aqueous solution of NaHCO₃ then brine, dried over MgSO₄, and concentrated *in vacuo*. The residue was purified by flash chromatography (silica gel, cyclohexane/ethyl acetate 10:1) to give acetate 13 as a colorless syrup (609 mg, 75%): [α]²³_{D} +9.4 (c 0.5, CHCl₃); ¹H NMR (400MHz, CDCl₃): δ 7.36 (m, 15 H, ArH) , 5.37 (td, 1H, *J*_{5,6} = 1.6 Hz, *J*_{6,7} = 6.1 and 7.2 Hz, H-6), 4.68-5.03 (m, 6H, ArCH₂), 4.63 (d, 1H, *J*_{1,2} = 3.6 Hz, H-1) , 4.02 (*J*_{2,3} = *J*_{3,4} = 9.2 Hz, H-3), 3.91 (dd, 1H, *J*_{4,5} = 10.2 Hz, *J*_{5,6} = 1.6 Hz, H-5), 3.82 (dd, 1H, *J*_{6,7} = 6.0 Hz, *J*_{gem} = 10.5 Hz, H-7), 3.73 (dd, 1H, *J*_{6,7} = 7.0 Hz, *J*_{gem} = 10.4 Hz, H-7), 3.67 (dd, 1H, *J*_{3,4} = 8.8 Hz, *J*_{4,5} = 10.2 Hz, H-4), 3.52 (dd, 1H, *J*_{1,2} = 3.5 Hz, *J*_{2,3} = 9.7 Hz, H-2), 3.41 (s, 3H, OMe), 0.89 (s, 9H, Si^{t}Bu), 0.02 (sx2, 6H, SiMe); ¹³C NMR (101MHz, CDCl₃): δ 170.0, 138.6, 138.1, 138.0, 128.4x3, 128.1, 127.9, 127.7, 127.6x2, 97.7, 82.3, 79.8, 78.2, 75.7, 74.6, 73.8, 73.3, 69.6, 61.4, 55.0, 25. 8, 21.1, 18.2, -5. 5x2; MS (DCI) : m/z 668 (M+NH₄); HRMS calcd for C₃₇H₅₄O₈NSi (MNH₄⁺): 668.3619. Found: 668.3615.

### Acetolysis of 13 (Scheme 3)

**1,2,3,4,6,7-Hexa-*O*-acetyl-6-D-*glycero*-D-heptoglucopyranose (14).** The procedure described for the preparation of compound 6 was followed. yield (83%). The α/β ratio was 1/0.09: ¹H NMR (400MHz, CDCl₃): δ 6.32 (d, *J*_{1,2} = 3.7 Hz, H-1 α), 5.69 (d, *J*_{1,2} = 8.0 Hz, H-1β), 5.44 (d, *J*_{2,3} = 10.1 Hz, *J*_{3,4} = 9.3 Hz, H-3α), 5.18 (ddd, *J*_{5,6} = 2.7 Hz, *J*_{6,7} *=* 4.3 and 7.0 Hz, H-6α), 5.17 (dd, *J*_{3,4} = 9.3 Hz, *J*_{4,5} = 10.5 Hz, H-4α), 5.05 (dd, *J*_{1,2} = 3.7 Hz, *J*_{2,3} = 10.3 Hz, H-2α), 4.34 (dd, *J*_{6,7} = 4.1 Hz, *J*_{gem} = 11.9 Hz, H-7α), 4.17 (dd, *J*_{4,5} = 10.5 Hz, *J*_{5,6} = 2.8 Hz, H-5α), 4.14 (dd, *J*_{6,7} = 7.3 Hz, *J*_{gem} = 11.9 Hz, H-7α), 2.02-2.18 (m, COCH₃); ¹³C NMR (101MHz, CDCl₃): δ 170.4, 170.1, 169.7, 169.6, 169.4, 168.5, 91.6 (C-1□), 88.7 (C-1β), 70.8, 69.8, 69.6, 68.9, 68.7, 61.3, 20.7, 20.6, 20.5, 20.4, 20.3; MS(DCI): m/z 480 (M+NH₄); HRMS calcd for C₁₉H₃₀O₁₃N (MNH₄⁺): 480.1717. Found: 480.1714.

### D-Glycal formation (Scheme 3)

**3,4,6,7-Tetra-*O*-acetyl-6-L-*glycero*-D-heptosylglycal (15).** The procedure described for the preparation of compound 7 was followed. yield (87%): [α]²³_{D} -20.9 (c 0.5, CHCl₃). ¹H NMR (400MHz, CDCl₃): δ 6.53 (dd, 1H, *J*_{1,2} = 6.3 Hz, *J*_{1,3} = 0.7 Hz, H-1) , 5.42 (td, 1H, *J*_{5,6} = 7.9 Hz, *J*_{6,7} = 2.8 and 4.6 Hz, H-6), 5.27 (ddd, 1H, *J*_{2,4} = 1. 1 Hz, *J*_{3,4} = 4. 6 Hz, *J*_{3,4} = 4.6 Hz, H-4) , 5.12 (tt, 1H, *J*_{2,3} = 5. 3 Hz, *J*_{3,4} = 4.5 Hz, *J*_{3,5} = 1.1 Hz, H-3) , 4.93 (qd, 1H, *J*_{1,2} = 6.0 Hz, *J*_{2,3} = 4.4 Hz, *J*_{2,4} = 1.2 Hz, H-2), 4.53 (dd, 1H, *J*_{6,7} = 2.8 Hz, *J*_{gem} = 12.3 Hz, H-7), 4.42 (qd, 1H, *J*_{3,5} = 1.3 Hz, *J*_{4,5} = 4.3 Hz, *J*_{5,6} = 8.4 Hz, H-5), 4.18 (dd, 1H, *J*_{6,7} = 4.7 Hz, *J*_{gem} = 12.3 Hz, H-7), 2.04-2.16 (s□4, 12H, COCH₃). ¹³C NMR (101MHz, CDCl₃): δ 170.6, 170.0, 169.5x2, 145.3, 98.5, 72.4, 67.6, 66.7, 64.5, 61.7, 20.9x2, 20.8, 20.7; MS(DCI): m/z 362 (M+ NH₄⁺) ; HRMS calcd for C₁₅H₂₄O₉N (MNH₄⁺): 362.1451. Found: 362.1452.

### Silylation of 15 (Scheme 3)

**3,4,6,7-Tetra-*O*-TBDMS-6-D-*glycero*-D-heptosylglycal (16).** The procedure described for the preparation of compound 11 was followed. yield (87%) : [α]²²_{D} -14.1 (c 0.5, CHCl₃); ¹H NMR (400MHz, CDCl₃): δ 6.37 (dd, 1H, *J*_{1,2} = 6.4 Hz, *J*_{1,3} = 0.8 Hz, H-1), 4.73 (dd, 1H, *J*_{1,2} = 6.4 Hz, *J*_{2,3} = 3.3 Hz, H-2), 4.35 (ddd, 1H, *J*_{5,6} = 8.7 Hz, *J*_{6,7} = 2.9 and 5.9 Hz, H-6), 4.13 (dt, 1H, *J*_{2,3} = 3.9 Hz, *J*_{3,4} = 4. 9 Hz, *J*_{3,5} = 0.9 Hz, H-3) , 4.03 (dd, 1H, *J*_{3,4} = 4.2 Hz, *J*_{4,5} = 5.9 Hz, H-4), 3.89 (td, 1H, *J*_{3,5} = 0.9 Hz, *J*_{4,5} = 5.9 Hz, *J*_{5,6} = 6.7 Hz, H-5), 3.78 (dd, 1H, *J*_{6,7} = 2.9 Hz, *J*_{gem} = 10.7 Hz, H-7), 3.66 (dd, 1H, *J*_{6,7} = 6.1 Hz, *J*_{gem} = 10.7 Hz, H-7), 0.93-0.95 (sx4, 36H, Si^{*t*}Bu), 0.09-0.16 (sx7, 24H, SiMe). ¹³C NMR (101MHz, CDCl₃): δ 143.3, 102.6, 80.4, 70.9, 70.8, 69.5, 64.3, 26.3, 26.0x2, 25.9. 18.4x2, 18.2, 18.1, - 3.3, -3.9, -4.0, -4.1, -4.3, -4.4, -5.4, -5.5; MS(FAB): m/z 655 (M+Na⁺); HRMS calcd for C₃₁H₆₈O₅Si₄Na (MNa⁺): 655.4042. Found: 655.4053.

### Nonstepwise fluorophosphorylation of 16 (Scheme 3)

**2-Deoxy-2-fluoro-3,4,6,7-tetra-*O*-TBDMS-6-D-*glycero*-β-1-(di benzylphosphoryl)-D-hepto-glucopyranoside (17).** The procedure described for the preparation of compound **12** was followed. yield (53%): [α]²²_{D} +2.6 (c 0.5, HC1₃) ; ¹H NMR (400MHz, CDCl₃): δ 7.38 (m, 10H, ArH) , 5. 46 (ddd, 1H, *J*_{1,2} = 7.4 Hz, *J*_{1,F} = 3.6 Hz, *J*_{1,P} = 7.4 Hz, H-1) , 5. 12-5. 18 (m, 4H, ArCH₂), 4.14 and 4.26 (t□2, 1H, *J*_{1,2} = 7.3 Hz, *J*_{2,3} = 7.3 Hz, *J*_{2,F} = 50.1 Hz, *J*_{2,P} = 3.3 Hz, H-2), 4.06 (ddd, 1H, *J*_{5,6} = 1.6 Hz, *J*_{6,7} = 5.5 and 7.4 Hz, H-6), 3.93 and 3.97 (tx2, 1H, *J*_{2,3} = 7.7 Hz, *J*_{3,4} = 8.0 Hz, *J*_{3,F} = 16.0 Hz, H-3), 3.89 (t, 1H, *J*_{3,4} = *J*_{4,5} = 8.0 Hz, H-4) , 3.72 (dd, 1H, *J*_{6,7} = 7.7 Hz, *J*_{gem} - 9.7 Hz, H-7), 3.59 (m, 2H, H-5 and H-7), 0.90-0.96 (s□4, 36H, Si^{t}Bu), 0.04-0.19 (sx7, 24H, SiMe) . ¹³C NMR (101MHz, CDCl₃): δ 128.5, 128.4x2, 127.9, 127.8, 96.4x2, 96.2x2, 93.5, 93.4, 91.6, 91.5, 77.3, 77.1, 76.7, 72.0, 71.9, 71.3, 69.4, 69.3x3, 26.2, 26.0, 25.9x2, -2.5, -3.2, -3.3, -3.4x2, -3.5, -3.7, -4.0. ¹⁹F NMR (235MHz, CDCl₃): δ 191.5 (dd, *J*_{2,F} = 50.0 Hz, *J*_{3,F} = 14.0 Hz). ³¹P NMR (101MHz, CDCl₃): δ -2.79 (s); MS (FAB) : m/z 951 (M+Na⁺) ; HRMS calcd for C₄₅H₈₂O₉FSi₄PNa (MNa⁺): 951.4665. Found: 951.4662.

### Deprotection of 17 (Scheme 3)

**2-Deoxy-2-fluoro-6-D-*glycero*-β-1-phosphoryl-D-heptoglucopy ranoside disodium salt (18) .** To a suspension of fluorophosphate 12 (69.1 mg, 0.074 mmol) and Pd/C (20 mg) in ethanol (5 mL) was added triethylamine (15 µL, 0.112 mmol). The mixture was hydrogenated under H₂ atmosphere for 1 h, and filtered through a pad of Celite. The filtrate was concentrated in vacuo to afford a triethylammonium salt, that was dissolved in tetrahydrofuran (5mL). To this solution was added tetrabutylammonium fluoride (100 mg, 0.318 mmol), and the mixture was stirred at room temperature for 1.5 h. After concentration in vacuo, to the residual syrup was added water (5 mL), thus generating a colorless precipitate. The precipitate was removed by filtration, and the aqueous solution was concentrated in vacuo. The resulting yellow syrup was dissolved in water (1mL), and passed through a Dowex 50Wx8 column (Na⁺ form, 2x20 cm). This solution was concentrated and the residue was dissolved in water (2mL), and the solution was lyophilized to afford a monophosphate 18 (18.9 mg, 78%) as a white powder: [α]²²_{D} +1.3 (*c* 0.5, H₂O); ¹H NMR (400 MHz, D₂O): δ 5.07 (ddd, 1H, *J*_{1,2} = 7.9 Hz, *J*_{1,F} = 2.5 Hz, H-1), 4.03 and 4.16 (ddx2, 1H, *J*_{1,2} = 7.9 Hz, *J*_{2,3} = 8.9 Hz, *J*_{2,F} = 50.9 Hz, H-2), 4.01 (m, 1H, H-6), 3.77 and 3.80 (tx2, 1H, *J*_{2,3} = *J*_{3,4} = 9.0 Hz, *J*_{2,F} = 14.8 Hz, H-3), 3.75 (m, 2H, H-7), 3.61 (dd, 1H, *J*_{4,5} = 10.1 Hz, *J*_{5,6} = 2.8 Hz, H-5), 3.54 (t, 1H, *J*_{3,4} = *J*_{4,5} = 10.1 Hz, H-4); ¹³C NMR (101 MHz, D₂O): δ 95.4□2, 95.1□2, 93.7, 93.6, 91.9, 91.8, 77.2, 74.7, 74.5, 71.5, 70.3, 70.2, 61.7; ¹⁹F NMR (235MHz, D₂O): δ -199.2 (dd, *J*_{2,F} = 51.7 Hz, *J*_{3,F} = 14.1 Hz); ³¹P NMR (101MHz, D₂O): δ 0.83 (s); HRMS calcd for C₇H₁₃O₉FP (M + H) 291.0214, found 291.0283.

### Synthetic procedures for the preparation of adenosine 5'-(β-D-manno-hexopyranosyl)-diphosphate triethylammonium salt 19 (scheme 4)

The synthesis starts with known β-phosphate mannoside **20** (Sabesan and Neira, 1992), that was prepared owing to procedures previously described (Sabesan and Neira, 1992 ; Zamyatina et al, 2003). The phosphate deprotection of 20 was carried out by catalytic hydrogenation using platinum oxide in methanol with a quantitative yield. Subsequent free phosphate 21 was subjected to AMP-coupling with AMP-morpholidate in pyridine at room temperature in the dark. After flash chromatography on silica gel, the ADP-mannoside 22 was isolated with a 26% overall yield. Tetra-acetate 22 was eventually deacetylated with triethylamine at 0°C during two days with a 83% yield.

### Phosphate deprotection

**2,3,4,6-tetra-O-acetyl-β-D-manno-hexopyranosyl-phosphate triethylammonium salt (21)**. To a solution of phosphate 20 (166 mg, 286 µmol, 1.0 eq.) in anhydrous MeOH (41 mL) under argon atmosphere, was added PtO₂ (55 mg). The reaction mixture was then placed under hydrogen atmosphere for 7h30. The mixture was filtered over Celite® , NEt₃ (60.3 µL, 429 µmol, 1.5 eq.) was added and the filtrate was concentrated *in vacuo* to give the deprotected phosphate 21 (161 mg, quantitative) as a triethylammonium salt (0.13 eq.) which was used without further purification. ¹H-NMR (400 MHz, D₂O) δ 5.54 (d, *J*₂₋₃ = 2.8 Hz, 1H, H-2), 5.45 (bd, *J*_{1-P} = 8.4 Hz, 1H, H-1), 5.36 (dd, *J*₂₋₃ = 2.8 Hz, *J*₃₋₄ = 10.1 Hz, 1H, H-3), 5.24 (dd, *J*₃₋₄ = 10.1 Hz, *J*₄₋₅ = 9.6 Hz, 1H, H-4), 4.46 (ABX, *J*₅₋₆ₐ = 3.3 Hz, *J*_{6a-6b} = 12.6 Hz, 1H, H-6a), 4.23 (ABX, *J*_{5-6b} = 1.2 Hz, *J*_{6a-6b} = 12.6 Hz, 1H, H-6b), 4.17 (ddd, *J*₄₋₅ = 9.6 Hz, *J*₅₋₆ₐ = 3.3 Hz, *J*_{5-6b} = 1.2 Hz, 1H, H-5), 3.21 (q, *J*_{CH2-CH3} = 7.3 Hz, 5.04 H, CH₂, NEt₃), 2.27 (s, 3H, CH₃, Ac), 2.15 (s, 3H, CH₃, Ac), 2.12 (s, 3H, CH₃, Ac), 2.04 (s, 3H, CH₃, Ac) , 1.28 (t, *J*_{CH2-CH3} = 7.3 Hz, 7.56H, CH₃, NEt₃); ¹³C-NMR (100 MHz, D₂O) δ 174.1, 173.7, 173.5, 173.0 (CO, Ac), 93.6 (C-1), 72.1, (C-5), 71.7 (C-3), 70.7 (d, *J*_{2-P} = 6. 1 Hz, C-2), 65.9 (C-4), 62.3 (C-6), 47.0 (CH₂, NEt₃) , 20.5 (3 x CH₃, Ac), 8.6 (CH₃, NEt₃) ; ¹H-non-decoupled ¹³C-NMR (100 MHz, D₂O) *J*_{C1-H1} = 162 Hz; ³¹P-NMR (101 MHz, MeOD) δ - 0.61 (s, OP(O)(OH)(OHNEt₃)); MS (FAB-, NBA): m/z 153 (100%), 427 (25%) [M-H⁺], 319 (5%); HRMS calcd. for C₁₄H₂₀O₁₃P: 427.0642 meas. 427.0646.

### AMP coupling

**Adenosine 5'-(2,3,4,6-tetra-O-acetyl-β-D-manno-hexopyrano syl)-diphosphate triethylammonium salt (22).** Using a rotary evaporator dried with toluene (2 x 50 mL) and flushed with argon, phosphate **21** (60 mg, 112 µmol, 1.0 eq.) was dried by repeated coevaporation with distilled pyridine (3 x 20 mL) and dried *in vacuo*. AMP-morpholidate (4'-morpholine-N,N'-dicyclohexylcarboxamidinium) (119.8 mg, 168.7 µmol, 1.5 eq.) was then dried by repeated coevaporation with distilled pyridine (3 x 20 mL) and dried *in vacuo.* To the dried phosphate 21, was added a solution of the dried AMP-morpholidate in distilled pyridine (20mL). The mixture was dried by coevaporation with distilled pyridine (2 x 20mL), dried *in vacuo* and pyridine (20mL) was then added. The reaction mixture was concentrated to remove about 65% of the solvent and was stirred in the dark at RT under argon atmosphere. After 115h, toluene was added and the mixture was concentrated *in vacuo.* Purification of the residue by flash column chromatography (silica gel: 22 g; CHCl₃:MeOH:NH₄OH (14%), 50:50,:2) gave fractions containing ADP-mannose which were combined and concentrated *in vacuo.* After solubilisation in water (40 mL), the mixture was cooled to 0°C and the pH was adjusted to 6 owing to Dowex 50W (H⁺ form) resin. After filtration over a PTFE filter (0.45 µm) and washing of the resin with water (3 x 2mL), the pH was adjusted to 7.5 with NEt₃ (3µL). Lyophilisation of the mixture afforded the ADP-mannose 22 (28.3 mg, 29 µmol, 26%) as a triethylammonium (2.1 eq.) salt. ¹H-NMR (400 MHz, D₂O) δ 8.53 (s, 1H, Ade), 8.24 (s, 1H, Ade), 6.14 (d, *J*_{1Rib-2Rib} = 5.8 Hz, 1H, H-1_{Rib}), 5.57 (bd, 1H, H-2_{Man}), 5.52 (bd, *J*_{1Man-P} = 9.5 Hz, 1H, H-1_{Man}), 5.18-5.14 (m, 2H, H-3_{Man}, H-4_{Man}), 4.74 (dd, *J*_{1Rib-2Rib} = 5.8 Hz, *J*_{2Rib-3Rib} = 5.3 Hz, 1H, H-2_{Rib}), 4.52 (dd, *J*_{2Rib-3Rib} = 5.3 Hz, *J*_{3Rib-4Rib} = 3.8 Hz, 1H, H-3_{Rib}), 4.42-4.37 (m, 1H, H-4_{Rib}), 4.33 (ABX, *J*_{5Man-6aMan} = 3.2 Hz, *J*_{6aMan-6bMan} = 12.6 Hz, 1H, H-6a_{Man}), 4.28-4.19 (m, 2H, H-5a_{Rib}, H-5b_{Rib}), 4.08 (ABX, *J*_{5Man-6bMan} = 2. 1 Hz, *J*_{6aMan-6bMan} = 12.6 Hz, 1H, H-6b_{Man}), 3.82-3.75 (m, 1H, H-5_{Man}), 3.20 (q, *J*_{CH2-CH3} = 7.3 Hz, 12.5H, CH₂, NEt₃), 2.23 (s, 3H, CH₃, Ac), 2.10 (s, 3H, CH₃, Ac), 2.07 (s, 3H, CH₃, Ac), 2.00 (s, 3H, CH₃, Ac), 1.28 (t, *J*_{CH2-CH3} = 7.3 Hz, 19. 1H, CH₃, NEt₃); ¹³C-NMR (100 MHz, D₂O) δ 173.9, 173.34, 173.32, 172.7 (CO, Ac), 158.1 (Cq_{Ade}), 152.4 (CH_{Ade}), 149.3 (Cq_{Ade}), 140.5 (CH_{Ade}), 118.8 (Cq_{Ade}), 93.9 (d, J_{1Man-P} = 3.4 Hz, C-1_{Man}), 87.3 (C-1_{Rib}), 84.2 (d, J_{4Rib-P} = 9.2 Hz, C-4_{Rib}), 74.8 (C-2_{Rib}), 72. 1 (C-5_{Man}), 71.4 (CH_{Man}), 70. 7 (C-3_{Rib}), 70.2 (d, J_{2Man-P} = 6. 8 Hz, C-2_{Man}), 65.7 (d, J_{5Rib-P} = 5. 1 Hz, C-5_{Rib}), 65. (CH_{Man}), 61.9 (C-6_{Man}), 47.0 (CH₂, NEt₃), 20.4, 20.3 (CH₃, Ac), 8.6 (CH₃, NEt₃); ¹H-non-decoupled ¹³C-NMR (100 MHz, D₂O) *J*_{C1-H1} = 165 Hz; ³¹P-NMR (101 MHz, D₂O) δ - 11.16 (d, Jp-p = 20.2 Hz), - 14.02 (d, Jp-p = 20.2 Hz) .

### Deacetylation

**Adenosine 5'-(β-D-manno-hexopyranosyl)-diphosphate triethy lammonium salt (19).** To a solution of tetra-acetate **22** (4.9 mg, 5.1 µmol, 1.0 eq.) in CD₃OD (567 µL) at 0°C, was added a solution of NH₄HCO₃ in D₂O (0.1 M, 729 µL) and NEt₃ (19 µL, 138 µmol, 27.0 eq.). After 50h at 0°C, the mixture was kept at 0°C, diluted in water (6 mL) and the pH was adjusted to 6.5 with Dowex 50Wx8 (H⁺ form) resin. The mixture was filtrated over a PTFE filter (0.45 µm), the resin was washed with water (3 x 2mL) and the filtrate was lyophilised. The residue was cooled to 0°C, diluted in water (4mL) and the pH was adjusted to 7.5 with Dowex 50Wx8 (H⁺ form) resin. Filtration over a PTFE filter (0.45 µm), washing of the resin (3 x 2mL) and lyophilisation of the filtrate afforded ADP-mannose 19 (3.2 mg, 4.3 µmol, 83%) as a triethylammonium (1.6 eq.) salt. ¹H-NMR (250 MHz, D₂O) δ 8.32 (s, 1H), 8.07 (s, 1H), 5.95 (d, *J* = 6.0 Hz, 1H), 5.05 (d, *J =* 8.5 Hz, 1H), 4.34 (dd, *J =* 5.0 Hz, *J* = 3.5 Hz, 1H), 4.24-4.17 (m, 1H), 4.07-4.00 (m, 2H), 3.89 (d, *J* = 3.0 Hz, 1H), 3.69 (dd, *J =* 12.0 Hz, *J =* 2.0 Hz, 1H), 3.73-3.43 (m, 3H), 3.35 (dd, *J* = 9.5 Hz, *J =* 9.5 Hz, 1H), 3.26-3.14 (m, 1H), 3.00 (q, *J*_{CH2-CH3} = 7.5 Hz, 9.6H, CH₂, NEt₃), 1.08 (t, *J*_{CH2-CH3} = 7.5 Hz, 14.3H, CH₃, NEt₃); ³¹P-NMR (101 MHz, D₂O) δ - 11.31 (d, *J*_{P-P} = 19.7 Hz), - 13.20 (d, *J*_{P-P} = 19.7 Hz).

### Screening assay development for protein WaaC and WaaF

The identified targets correspond to protein WaaC and F and are involved in lipopolysaccharide biosynthesis in Gram negative bacteria.

Enzymatic activity of these proteins was previously reported using cell extract to provide for the donor substrate which is not commercially available sugar nucleotide ADP-L-glycero-β-D-manno-heptose. It is provided by a crude extract containing compounds of molecular weight lower than 3000 daltons. The acceptor substrate described was either ReLPS or synthetic analogues of lipid A and KdO. (Kadrmas and Raetz 1998 ; Gronow et al. 2000). The products of the reaction were detected either by monoclonal antibodies able to recognize heptose or by thin layer chromatography. All these methods to detect heptosylation reaction were not quantitative and not amenable for a screening assay.

According to the invention, the heptosyltranferase activity for both proteins WaaC and WaaF is measured. It is a high-throughput assay based on fluorescence detection of an end product of the reaction.

The donor substrate is a non commercially available sugar nucleotide ADP-L-glycero-α-D-manno-heptose. Said nucleotide sugar was synthesized as described by Zamyatina et al, 2003.

The acceptor substrate is the Re-LPS composed of the lipid A linked with the two Kdo. Said acceptor is commercially available.

The corresponding enzymes are produced as recombinant proteins after cloning of the genes according to methods well known from the one skilled in the art.

To eliminate residual LPS contaminants from the enzyme extracts, a mutated strain of E.coli BL21 was constructed to express the recombinant enzymes.

The deletion of both genes corresponding to heptosyltransferases I and II was performed in *E.coli* BL21 using a single mutagenesis step as described in example 1 of WO 03/074553. This double mutant strain was then used to express the recombinant enzyme by transformation with the corresponding expression vector.

The purification was carried out under native conditions and the biological activity of the purified proteins was assessed by analysis of the reaction products using tricine SDS-PAGE.

A fluorimetric *in vitro* assay was set up to quantify the enzymatic activity of purified heptosyl transferase.Said fluorimetic assay is used to identify inhibitors and for measuring IC₅₀ determinations.

WaaC IC₅₀ assays are performed in black 96-well plates (Wallac) in a final volume of 100µL at pH 7.0 comprising: the inhibitor studied dissolved in 2µL DMSO at 10 different concentrations, 50mM HEPES, 10mM MgCl₂, 50mM KCl, 0.1% Triton-), 3µM Re-LPS (Sigma, purified from Salmonella Minnesota), 1nM of purified WaaC, 5u/mL Pyruvate kinase (Sigma), 50µM Phosphoenolpyruvate (Sigma), 5u/mL Lactate dehydrogenase (Sigma) and 1.5µM NADH (Sigma). Fluorescence intensity of NADH (λₑₓ= 360nm, λₑₘ=520nm) is read once before enzyme addition (background), and is followed kinetically afterwards. Initial velocities are determined from the linear phase after background substraction. IC₅₀ curves are fitted to a classical sigmoidal model with Hill slope.

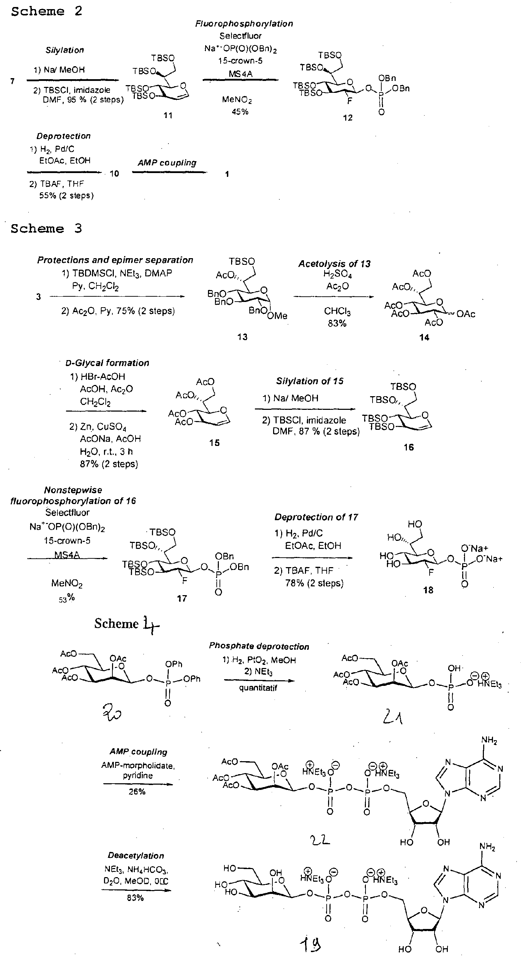

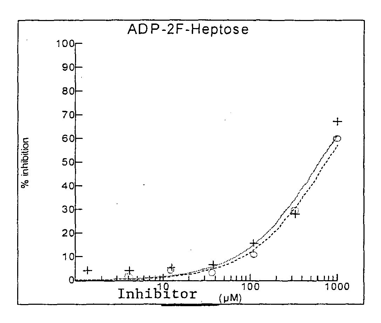

Using this assay, it was possible to measure inhibitory activity of the 2-fluoro 2deoxy heptose derivative of the substrate on Waac enzymatic activity with an IC₅₀ = 57 µM (figure 2).

### BIBLIOGRAPHIC REFERENCES

- Heinrich et al., 1998, Mol. Microbiol. 30:221-232
- Valvano et al., 2000, J. Bacteriol., 182:488-497
- Kneidinger et al., 2001, J. Biol Chem 276:20935-20944
- Kneidinger et al., 2002, J. Bacteriol., 184:364-369
- Jarosz and Kozlowska, Pol. J. Chem. 1996, 70, 45-53
- Sabesan and Neira, Carbohydr. Res. 1992; 223, 169-185
- Zamyatina et al, 2003
- Kadrmas and Raetz 1998, *J. Biol. Chem.* **273,** 2799-2807
- Gronow et al. 2000, *Eur. J. Biochem.* **267**, 6602-6611

## Claims

1. A method for making osyl and hexoses derivatives of formula 1, especially 2-fluoro-2-deoxy derivatives. 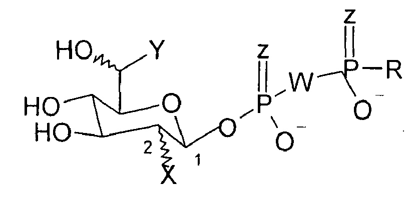 wherein
- R is a nucleoside such as adenosine, cytidine, guanosine, uridine and deoxy analogs such as 2-deoxy,
- X represents OH, halogen, particularly F, NH₂
- Y represents H, CH₂OH, CH₂NH₂, CH₂OPO₃, CH₂OSO₃
- Z represents O or S, and
- W represents O,NH, or CH₂,
said method comprising the steps of:
a) stereoselective fluorophosphorylation of tetrapivaleate 8 to give β-gluco-type fluorophosphate 9,
b) hydrogenation and deprotection of 9 to give a monophosphate 10,
c) coupling 10 with (R)P-morpholidate to give crude sugar nucleotide 1, or alternatively,
d) deacetylation then silylation of heptoglycal 7 to give tetrasilylated glycal 11,
e) fluorophosphorylation of 11 to give β-gluco type fluorophosphate 12,
f) deprotection of 12 to give 10 and coupling radical R to give 1.

2. The method of claim 1, wherein tetrapivaleate 8 is obtained by using the following steps:
g) dihydroxylation of olefin 2 to obtain diol 3 as a mixture of two diastereomers, D and L-glycero-heptoses,
h) regioselective silylation at OH-7 giving 4,
i) triflation at OH-6, and inversion at C6 to give L-glycero type silyl ethers 5L and 5D,
j) removal of the protecting groups of 5 by acetolysis to give per - 0 - acetate 6,
k) treating acetate 6 to give acetylate heptoglycal 7, and either
l) converting 7 into tetrapivaleate 8.

3. The method of claim 1, wherein dihydroxylation step **g** is carried out with osmium tetroxide and NMO in acetone and water, giving a mixture of two diastereomers D and L-glycero heptoses, with D-glycero sugar as major component.

4. The method of claim 2 or 3, wherein, to obtain the L-glycero type sugar as major component, a regioselective silylation at OH-7 with TBD MSCl, in presence of NEt₃ and DMAP is performed according to step **h** and triflation at OH-6 is carried out by treatment with Tf₂O.

5. The method of anyone of claims 2 to 4, wherein the inversion at C-6 according to step **i** is carried out with cesium acetate in the presence of 18-crown-6 under ultrasound to afford two diastereomers 5L (major) and 5D (minor) from 4, which are optionally separated by standard procedures like on silica gel column chromatography.

6. The method of anyone of the claims 2 to 5, wherein the removal of all protecting groups of 5 of step **j** is carried out with excess sulfuric acid in chloroform and acetic anhydride giving *per*-*O*-acetate 6.

7. The method of anyone of claims 2 to 6, wherein the glycal formation of step **k** is performed by subjecting acetate 6 to bromination, followed by treating with zinc powder, copper sulfate, and sodium acetate in acetic acid and water, giving an acetylated heptoglycal 7.

8. The method of anyone of claims 2 to 7, wherein, according to step 1, heptoglycal 7 is converted into tetrapivaloate 8 by Zemplen's deacetylation and *per-O-pivaloylation* with pivaloyl chloride.

9. The method of anyone of the preceding claims, wherein the stereoselective fluorophosphorylation step is performed according to step **a** by treating tetrapivaloate 8 with Selectfluor™ (triflate form), sodium dibenzylphosphate, and 15-crown-5 in nitromethane to give β-gluco-type fluorophosphate 9.

10. The method of anyone of the preceding claims, wherein pivaloylated β -fluorophosphate 9 is subjected to hydrogenation, then depivaloylation by tetrabutylammonium hydroxide to afford monophosphate 10 in quantitative yield according to step **b**.

11. The method of anyone of the preceding claims, wherein the coupling step **c** is carried out by reacting monophosphate 10 with (R)P-morpholidate in the presence of 1*H*-tetrazole in pyridine at room temperature to give crude sugar-nucleotide 1.

12. The method of anyone of claims 9 to 11, wherein according to step **d**, heptoglycocal 7 is deacetylated following a standard Zemplen's procedure then silylated to furnish tetrasilylated glycal **11.**

13. The method of claim 12, wherein the fluorophosphorylation of step **e** gives the β -gluco-type fluorophosphate 12 along with its isomeric α-gluco-type fluorophosphates.

14. The method of claim 12 or 13, wherein fluorophosphate 12 is deprotected according to step **f** by hydrogenation and treatment with TBAF in THF to afford monophosphate 10 in yield above 50%.

15. The method of anyone of the preceding claims, wherein R is adenosine.

16. A method for making 2-fluoro-L-glyceroheptose derivatives 1 *via* a biomimetic chemo-enzymatic synthesis, wherein ADPH, D-glycero-D-manno-heptose- β -1- phosphate is transformed into a nucleotide-sugar through an enzymatic AMP transfer (catalyzed by RfaE).

17. The method of claim 16, wherein fluorinated analogs of D-glycero-D-manno-heptose- β -1-phosphate, such as the fluorophosphate 18 are used as a substrate of_ RfaE, the reaction leading to the formation of an intermediate ADP-fluoroheptose which enzymatically epimerized by WaaD to generate the target molecule 1.

18. The method of claim 17, wherein the synthesis of 18 comprises protections at C6 and C7, acetolysis, glycal formation, fluorophosphorylation of a silylated intermediate and deprotections.

19. The method of anyone of the preceding claims, wherein the following derivatives A, B, C, D, and E are obtained 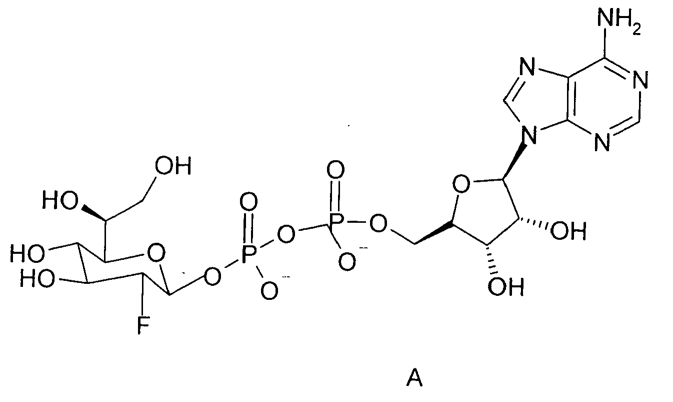 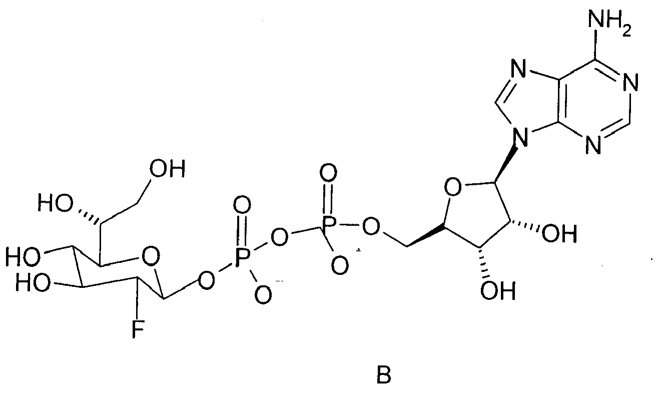 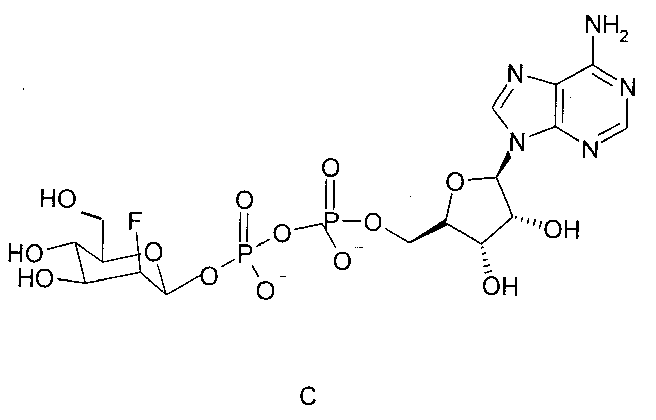 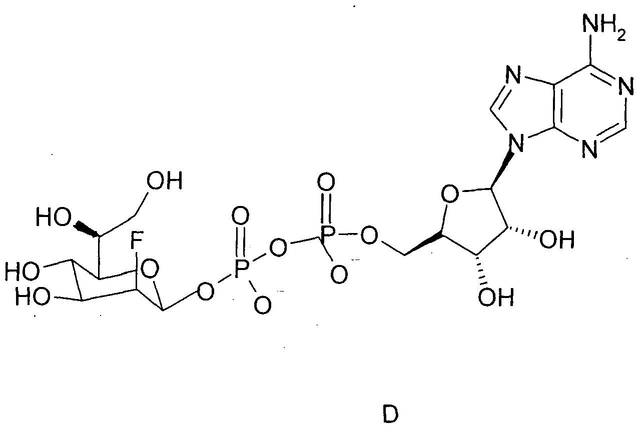 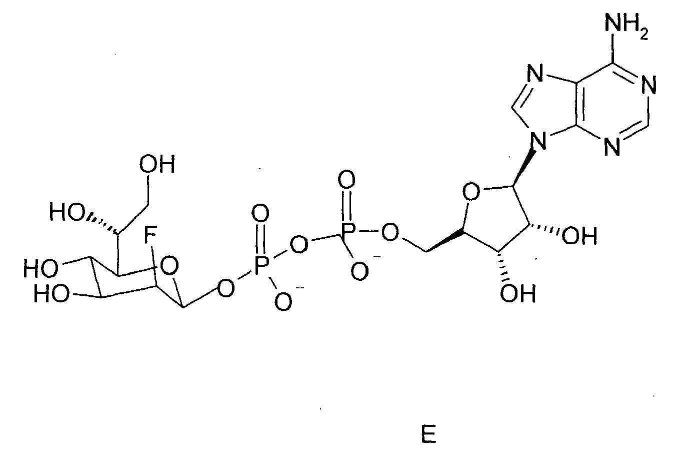 and alpha corresponding anomers.

20. The derivatives of formula 1, wherein Y represents CH₂OH, X represents F, and the other substituents have the meanings given in claim 1.

21. The use of a derivative such as obtained by the method according to anyone of claims 1 to 19,or according to claim 20 as inhibitors of heptosyl tranferase proteins of pathogenic bacteria.

22. The use of adenosyl 2-fluoro heptose as inhibitor of waaC.

23. A quantitative assay for determining heptosyltransferase activity of target proteins, comprising incubating, as donor substrate, an ose derivative such as obtained by the method according to anyone of claims 1 to 19,or according to claim 20, particularly ADP-L-glycero-α-D-manno-heptose and, as acceptor substrate, Re-LPS, composed of lipid A with two Kdo, and measuring the reaction products of transferase activity using a fluorimetric assay.
